# EUROPEAN PATENT SPECIFICATION

(11) **EP 0 623 004 B1**
(45) Date of publication and mention of the grant of the patent: **02.05.1997**
(21) Application number: 93901858.6
(22) Date of filing: 13.01.1993
(51) Int. Cl.: A61B 17/02, A61B 17/00

(54) **SURGICAL INSTRUMENTS**
CHIRURGISCHE INSTRUMENTE
INSTRUMENTS CHIRURGICAUX

(30) Priority: 21.01.1992 GB 9201214; 30.07.1992 GB 9216233
(43) Date of publication of application: 09.11.1994
(73) Proprietor: McMAHON, Michael John, Leeds, West Yorkshire LS16 5PD (GB); MORAN, Peter, Leeds, West Yorkshire LS13 4RN (GB)
(72) Inventor: McMAHON, Michael John, Leeds, West Yorkshire LS16 5PD (GB); MORAN, Peter, Leeds, West Yorkshire LS13 4RN (GB)
(74) Representative: Sherrard-Smith, Hugh
(86) International application number: GB9300064
(87) International publication number: WO9313713

(56) References cited:
- DE-A- 4 021 153
- US-A- 4 239 036
- US-A- 4 754 909

## Description

The present invention relates to surgical instruments.

In abdominal surgery it is possible for a surgeon to examine or operate on abdominal organs without the physical insertion of the surgeons fingers. This is done by making a hole through the skin and other tissues in order to enter a body cavity, blowing inert gas under pressure into the abdominal cavity through that hole to create space, and inserting an endoscopic camera through that opening so that the surgeon can view the organs on a remote monitor. Other tubes are then placed in the abdominal wall (usually of 5 - 11 mm diameter), which are sealed to prevent or restrict the egress of the inert gas, and instruments can then be inserted through these tubes. The instruments can either be for operating on the organs or for pushing organs out of the way, and the surgeon is able to manipulate them from outside of the body.

As the tubes are of restricted diameter (usual maximum 11 mm) all that can be inserted through the tubes is a relatively straight instrument having a diameter of slightly less than the tube through which it is inserted. Thus, where a surgical retractor is used, the surgeon must displace organs and retain them in a displaced position with the use of a straight rod. It is difficult to so displace and retain the organs with such a straight rod and the organs are prone to slipping over the end of the rod thereby impeding the operation.

The surgical instruments described herein aim to reduce the problems associated with this type of surgery.

U.S. 4 754 909 (Barker) shows a stapler including a plurality of rigid spine segments each having a concave end surface and an opposite convex end surface. A stiffening assembly extends through the segments and can be tightened, when the segments are in the desired position, to hold the segments in that position.

According to the present invention, a surgical instrument includes an elongate portion arranged, in use, to be inserted through a restricted opening into a body cavity, the elongate portion comprising a plurality of segments movable relative to each other from a first to a second position whereby, when movement of the segments occurs from the first to the second position the relative direction in which a part of the elongate portion extends when compared to another part of the elongate portion is altered characterised in that, in the second position of the segments, further movement of the segments away from the first position is inhibited by abutments provided on the segments.

Further movement of the segments away from the first position may be inhibited by abutment of adjacent segments.

When the segments are urged towards each other from the first to the second position, the force urging the segments towards each other may act between two locations where a segment contacts an abutment.

The segments may be comprised by separate pieces.

The segments may be comprised by adjacent portions which are integral with each other and the segments may be integrally moulded. The segments may be formed by removing a portion of integral material.

The segments may be guided during at least part of their movement from the first to the second position.

Segments may cooperate with each other when in the second position to inhibit movement relative to each other in at least one direction transverse to the longitudinal extent of the elongate member.

At least three segments may be included movable relative to each other from the first position to the second position whereby movement of those segments from the first to the second position causes relative movement between an intermediate segment and an adjacent segment on one side to be in a first direction relative to the longitudinal extent of the elongate portion and relative movement between the intermediate segment and an adjacent segment on the other side to be in a second direction extending at an angle to both the first direction and the longitudinal extent of the elongate portion.

In the first position, the elongate portion may extend in a generally straight direction.

In the second position the elongate portion may curve through more than 45° or 90° or 120° or in the region of 180° or more.

In the second position, the elongate portion may form a constriction.

The segments may be arranged to be biased towards each other in order to move from the first to the second position and the segments may be biased towards each other by a flexible member. The flexible member may extend through the segments. The flexible member may be arranged to be caused to be tensioned by control means located outside of a body when, in use, the segments are located within a body.

The cross sectional area of the instrument, in a direction perpendicular to the extent of the longitudinal portion, may remain largely unchanged along the longitudinal extent in both the first and second positions.

Segments may be located at an intermediate portion along the elongate portion.

Segments may be located towards the end of the elongate portion.

An extent of the elongate portion may be covered with a protective layer. The extent of the elongate portion which is covered by the protective layer may be substantially the complete extent.

A mechanism may be included connected to one of the segments on the elongate portion.

Substantially the complete extent of the elongate portion may be comprised by segments movable relative to each other from the first to the second position.

An elongate resilient member may be arranged to act on adjacent segments in order to urge them towards a particular position. The resilient member may be arranged to urge adjacent segments towards the first position. The resilient member may be arranged to enable the instrument to have sufficient stiffness to enable use at a position in which the segments are away from the first position, in a second position which is away from a limit position.

The present invention may be carried into practice in various ways, but various embodiments will now be described by way of example and with reference to the accompanying drawings, in which:
Figure 1 is a side view of a first embodiment of a surgical instrument comprising a surgical retractor 1 with an end 2 in a straight configuration;
Figure 2 is a view of the end 2 of the retractor shown in Figure 1 in a straight hook configuration;
Figure 3 is a view of an end 2 of a retractor similar to that shown in Figure 1 in an angled hook configuration;
Figure 4 is a schematic perspective view of one of the segments 3 at the end 2 of the retractor shown in Figure 1;
Figures 5A and 5B are a front view and a front sectional view respectively of a surgical retractor 4 with an end 5 in an extended position;
Figure 6 is an enlarged discontinuous view showing the blade joints of the end 5 of the connector shown in Figures 5A and 5B;
Figures 7A and 7B are a side and end view respectively of one end of a retractor in a straight hook configuration;
Figure 8 is a side view of a part of a retractor having an angled section at an intermediate extent of its length;
Figure 9 is a side view of an end of a retractor having a gentle curve;
Figures 10A and 10B are an underneath and a side view respectively of a retractor having an angled hook configuration;
Figures 11A, 11B, 11C and 11D are side, plan and end views respectively of a "change over" segment included in the retractor shown in Figures 10A and 10B;
Figures 12A and 12B are side and perspective views of two of the segments shown in the retractor of Figures 10A and 10B;
Figure 13 is a perspective view of a double fork retractor or pusher;
Figure 14 is a perspective view of a constricting hook;
Figure 15 is a schematic perspective view of a segment in a retractor showing the location of a rigid wire spring;
Figure 16 is a perspective view of a retractor including a straight hook at its end but also having a bent portion at a mid extent;
Figure 17 is a perspective view of a retractor similar to that shown in Figure 16 with the exception that the intermediate bent portion can be straight during insertion and removal of the retractor;
Figure 18 is a schematic side view of an elongate support member holding a part of a person's body raised up in order to avoid the need for inert gas when performing an operation or examination in a body cavity;
Figures 19, 20 and 21 are schematic perspective views showing the end of an instrument having scissors, a needle holder and gripping forceps located at the free end thereof;
Figure 22 is an end view of an instrument showing the forceps, needle holders or scissors facing back towards the direction into which they have been inserted into the body cavity;
Figures 23A and 23B are a perspective side view and a sectional view of an eye at the end of ligature insertion apparatus;
Figures 24, 25, 26, 27, 28 and 29 are views of further configurations which the end of the retractor may have, when in an activated position;
Figure 30 is a front view of a retractor with the end in an activated position;
Figures 31A, 31B and 31C are a side, front and end view of an end segment;
Figure 32 is a schematic perspective view of an alternative configuration for the retractor, and
Figure 33 is a schematic sectional view through the end of a retractor incorporating a memory metal core.

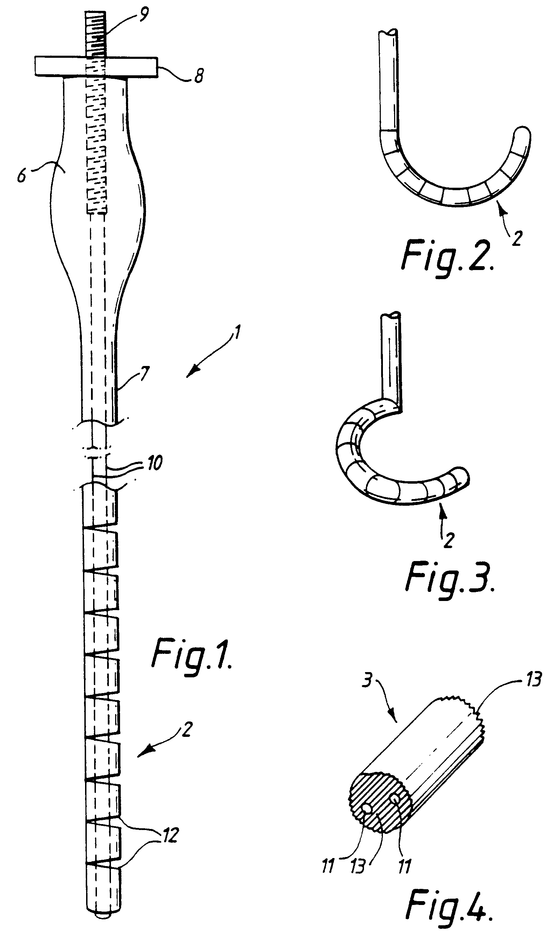

Figure 1 shows a retractor 1 having a handle 6 which is connected to the end 2 via a hollow rod 7. In use, with the end in the configuration shown in the drawing, the end 2 and part of the rod 7 are fed through a tube in the abdominal wall. The surgeon is then able to manipulate the retractor by the handle 6 and change the configuration of the end 2 into the straight hook shape shown in Figure 2 by rotating a knurled actuating nut 8.

The nut 8 is threadably connected to a screw member 9 whereby, when the nut 8 is rotated in a clockwise direction, looking from the free end of the handle, the screw member 9 is caused to move translationally away from the end 2. A loop of wire 10 is connected at its free ends to the member 9, and both sides of the loop pass through openings 11 in each segment 3. Accordingly as the wire 10 moves further into the rod 7 the segments are caused to tighten against each other.

As the segments 3 bear against each other they are caused to move out of the axial extent of the rod as the end faces 12 of each segment are formed at a slight angle to the perpendicular to the axis of the rod. In Figure 1 the upwardly facing surfaces of each segment are parallel with each other as are the downwardly facing surfaces. Accordingly adjacent faces come into abutment with each other as the wire is tightened, and they take up the configuration shown in Figure 2 in which a straight, substantially rigid hook which subtends approximately 180° is formed. Accordingly in the position shown in Figure 1, each face extends at an angle of approximately 10° to the axis of the tube.

In order for the segments to take up the shape shown in Figure 3, in which a substantially rigid hook which subtends approximately 180° in a direction generally at right angles to the axis of the rod, the face of at least one of the segments is angled differently. For instance, when the end 2 is in the relaxed position and extends generally in line with the axis of the rod 7, the uppermost segment faces the rod with a face extending at 45° to the axis of the rod, and the rod may be correspondingly angled at its end. Thus when the wire is tightened, the segment adjacent to the rod is caused to turn through 90°. The remaining upper and lower faces of the other segments may be parallel to each other in the configuration shown in Figure 1 as previously described.

The hooks shown in Figures 2 and 3 can be used quickly and conveniently to displace or hold the organs in the required position.

To release the segments from the configuration shown in Figures 2 or 3 the nut 8 is rotated in the opposite direction to release the tension in the wire. The wire is sufficiently strong, and the distance between the segments sufficiently small for the flexure of the wire to hold the segments generally straight for ease of insertion or removal when the hook configuration is not required. As the wire is threaded through two openings in each segment the strength of the wire and the close proximity of the segments prevents any significant relative turning of the segments around the longitudinal extent of the end 2.

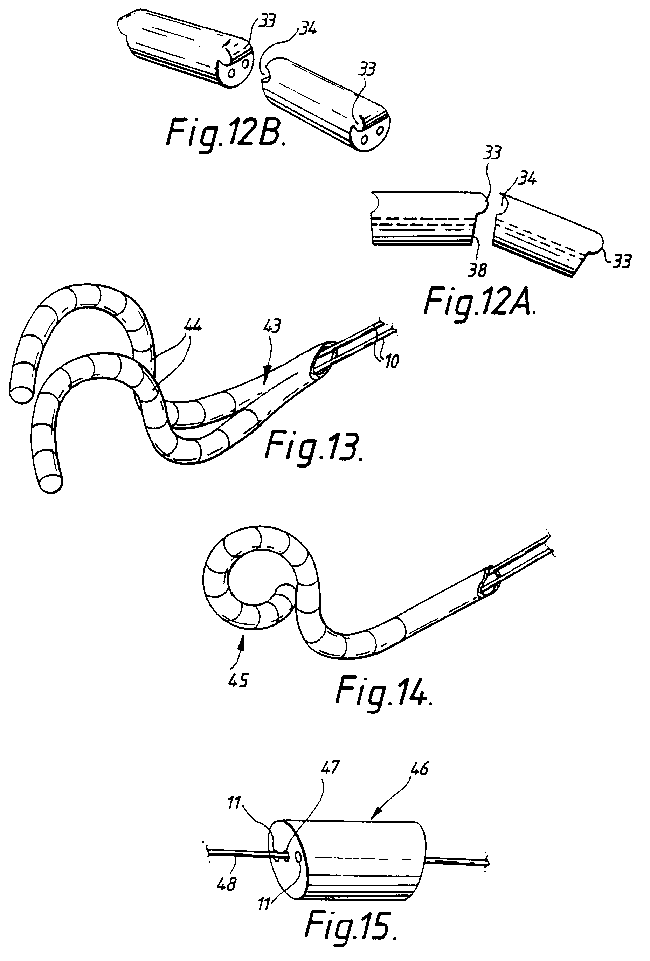

The face of each segment which is caused to abut against another part of the retractor when in the hook configuration is formed with styrations 13 which are parallel to each other and parallel to adjacent styrations such that co-operating faces do not tend to slip in a rotational or translational sense. Alternatively an arrangement as in Figure 12 can be used to restrain such movement.

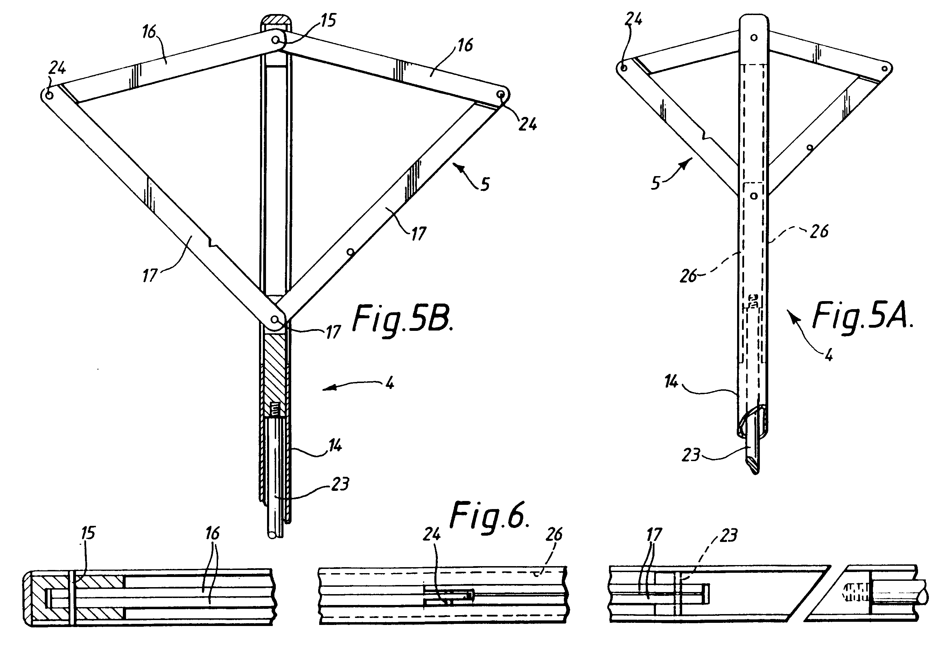

The retractor 4 shown in Figure 5 includes a shaft 14 which connects to an operating mechanism (not shown). The end 5 includes a linkage which, as shown in Figures 5A and 5B, includes opposed cross members 16 extending out at approximately 90° to the axis of the tube and a pair of supports 17 extending back along and into the shaft 14 from the proximal end of the respective supports 17. In the illustrated position, the retractor can be used in an abdominal cavity to displace or retain organs.

In order to move the retractor from the position shown in Figures 5A and 5B to a position in which the members 16 and the supports 17 lie within the area bounded by the shaft 14, an extension of an handle is used to slide a rod 23 away from the working end of the instrument.

The handle which can be pushed or operated by a lever mechanism from outside of a cavity within which the end 5 is located, is connected to a rod 23 which can slide within substantially the whole length of the shaft. When the rod 23 is slid within the shaft away from the working end, a pivot pin 23 at the end 5 is caused to move away from the working end. The pin 23 also extends through and pivotally connects each of the supports 17 and accordingly their ends are caused to move further into the shaft. Pivots 24 also connect the supports 17 to the members 16, and the members 16 are in turn connected by a pin 25 to the end of the shaft 14. Accordingly the members 16 pivot inwardly and the supports 17 slide and move in until they all lie substantially within the extent of the rod 14 in a "collapsed" condition.

In order for the members 16 and the supports 17 to be able to lie within the shaft 14, a slot 26 extends across the end of the shaft as shown in Figure 6. In the position shown in Figure 6, the member 16 and the supports 17 are in a collapsed position.

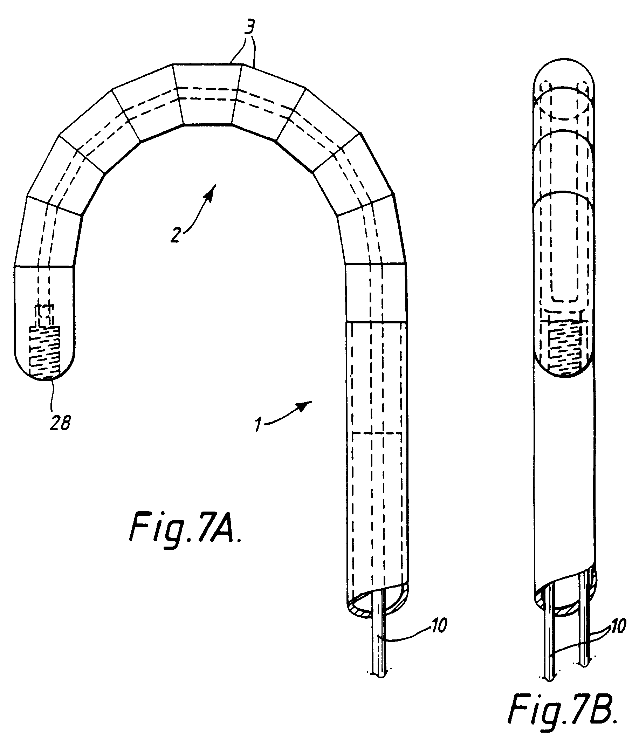

The embodiment shown in Figures 7A and 7B is similar to that shown in Figure 2 in that it comprises a retractor 1 having an end 2 movable from a straight configuration to the illustrated configuration shown upon effective tightening of both extents of the wire 10.

The end of the retractor is provided with a segment 27 which has a threaded opening 28 at its free end. The wire 10 can be manipulated into position through this opening during assembly of the retractor. That end can be closed by a threaded cap, if desired, to enable the device to present a smooth surface to the body during insertion.

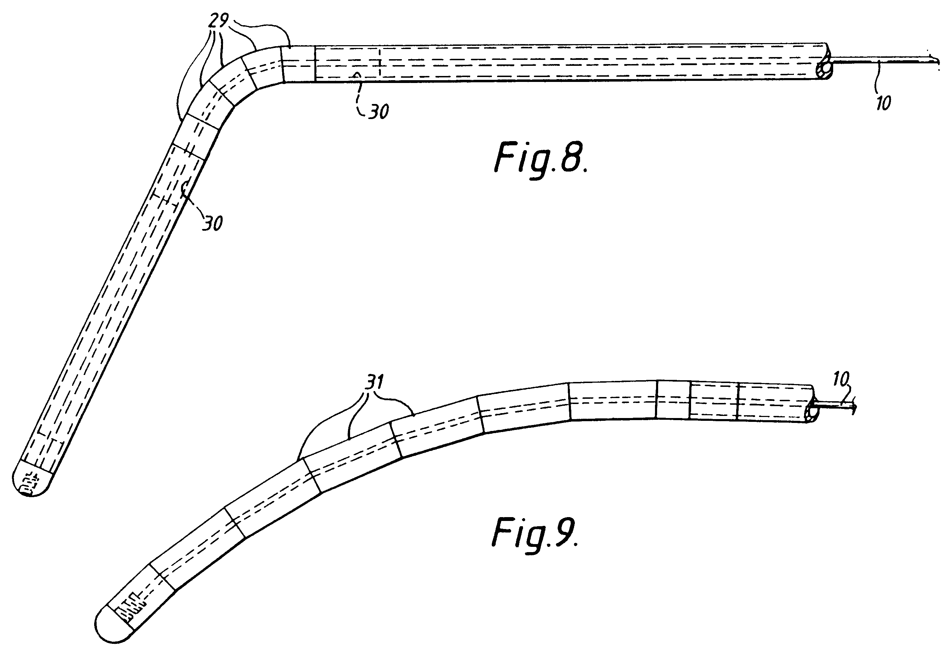

In Figure 8, the retractor is provided with five angled segments 29 at an intermediate extent along its extent. Accordingly, when the wire 10 is tightened, the segments take up the position shown. Then end segments in the row are both screwed into the adjacent straight sections along the threaded portion 30 shown.

In Figure 9, the segments 31 are of a larger extent than the other segments previously described as the retractor is only required to take up the fairly gentle curve shown when tightening the wire 10.

In Figures 8 and 9 and other figures, an instrument is shown which undergoes a change in direction in two dimensions only upon tightening of the wire. However, it will be appreciated that the instruments could be modified to undergo a change in direction in three dimensions. Similarly where instruments which undergo a change in direction in three dimensions are shown they could be modified to undergo a change in direction in two dimensions only.

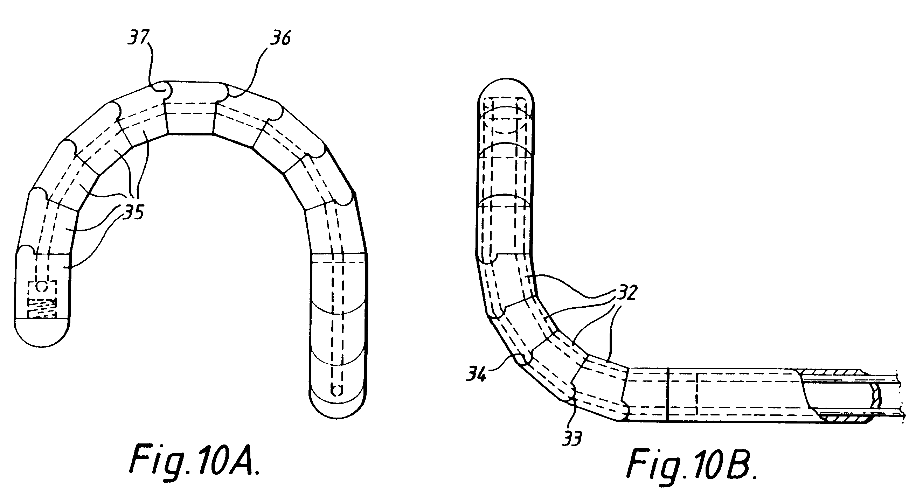

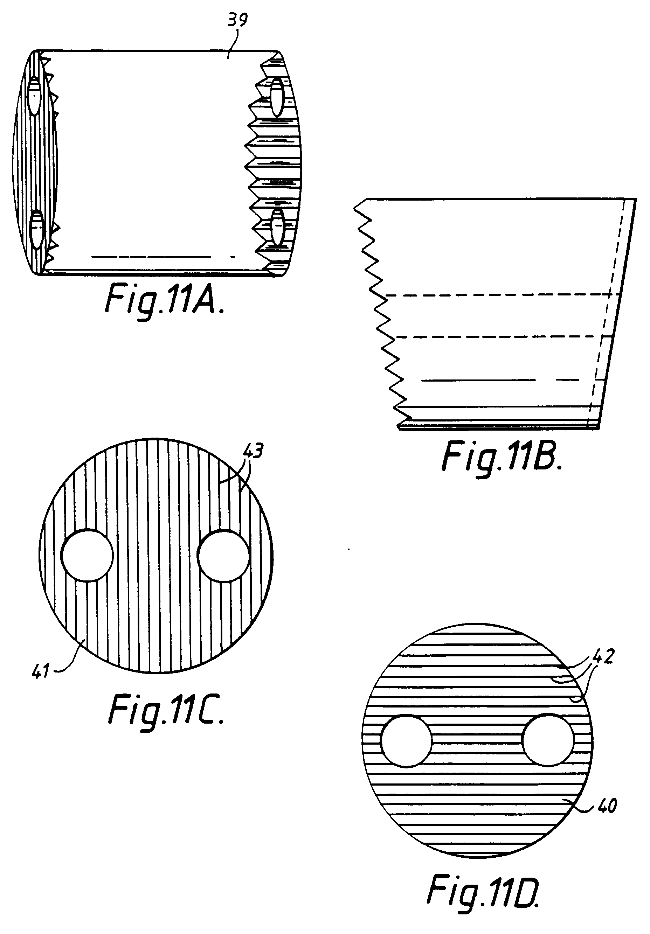

Figures 10A and 10B show an angled hook configuration for a retractor similar to that previously described in relation to Figure 3. The retractor is made up of an initial series of five segments 32 each including a rod section 33 at one end and a socket section 34 at the other end and a further series of segments 35 having a rod section 36 at one end and a socket section 37 at the other where they cooperate with adjacent segments.

Upon tightening of the wire, the retractor changes from a straight configuration to the curved configuration with each rod section 33 or 36 rocking in an adjacent socket section 34 or 37, as shown in more detail in Figures 12A and 12B, until their adjacent angled faces 38 are parallel with each other or abut each other.

The initial series of segments 32 have the longitudinal axes of the rod sections parallel with each other and are configured such that those sections undergo a change in extent along the retractor of 90°, as shown in Figure 10B. The remaining segments 35 have the longitudinal axes of their rod sections 36 parallel with each other but at right angles to the other section 33. Accordingly the segments 35 undergo a change in angle of 180° with those segments undergoing that change in a direction perpendicular to the change which has occurred for the segments 32. Thus it can be seen that, where the segments 32 and 33 meet, it is necessary for the socket of one of the segments 32 at one end thereof to be at right angles to the rod section at the other end.

Figures 11A, B, C and D show a segment 39 having end faces 40 and 41 respectively which extend at different angles to the longitudinal extent of the segment. The end face 40 has horizontally extending ridges and troughs 42 (when viewed from one side as in Figure 11D) and the other face 41 has vertically extending ridges and trough 43 (when viewed from the side as in Figure 11C). These end faces 40 and 41 are arranged, in use, to cooperate with adjacent segments having mating faces with ridges and troughs which extend in the same direction as the face which they are to mate with. Accordingly the segment 39 is used where a change in the direction in which the instrument is extending is desired.

The ridges and troughs described herein, and the rod sections and sockets described in relation to Figures 10A and 10B give adjacent segments stability in at least one relative direction of possible translational movement. The wire 10 also gives resistant to rotational and translational movement between the segments, when tightened.

Once the principle of being able to change the direction of a generally straight instrument is established, the bent configuration of the apparatus can be designed as required.

Figure 13 shows a double fork retractor or pusher 43 in which the tightening of a pair of single wires 10 (as illustrated) or double wires cause two rows of segments 44 to extend at an angle. The wire or wires 10 may act to pull the ends of pairs of wire extending up each row of segments 44 to pull the segments tight.

In the embodiment shown in Figure 14, the end of the instrument can be made to take up the form of a hook 45 in which the end turns back on itself and the end can be used to choke or grip a part of the body within the surrounded region shown.

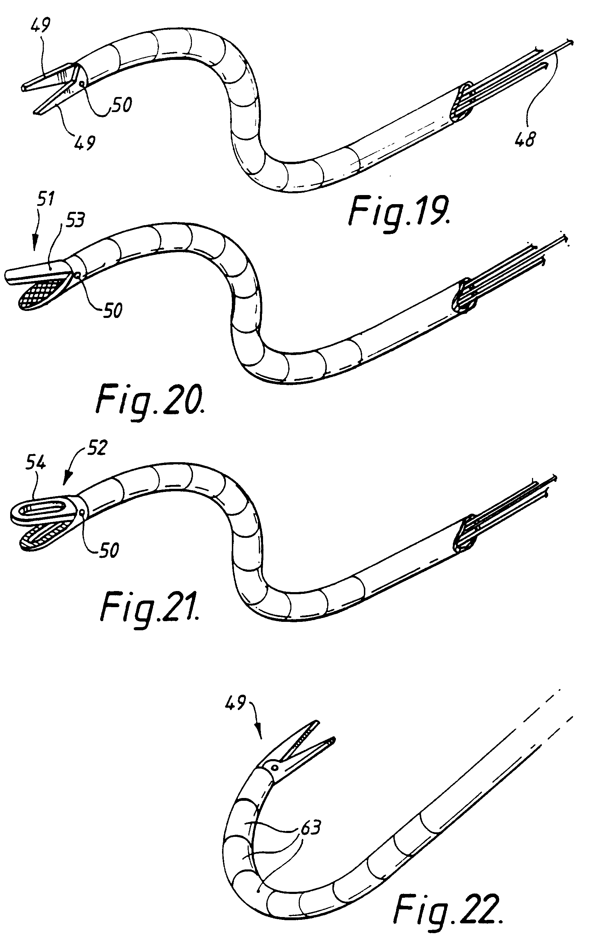

Figure 15 shows an angled segment 46 which can be used with any of the retractors or other instruments shown. The segment includes a central opening 47 through which a flexible element 48 passes. The element 48 may be used to import a degree of stiffness between adjacent segments so that when the tension in the conventional wire (not shown) which passes through the openings 11 is relaxed, the element 48 flexes the line of segments towards the position, or to the position in which they extend in a straight line. Alternatively or additionally, the element or filament 48 can be pulled through the openings 47 in aligned segments to operate a mechanism at the end of the instrument, as will be described later in relation to Figures 19 to 22.

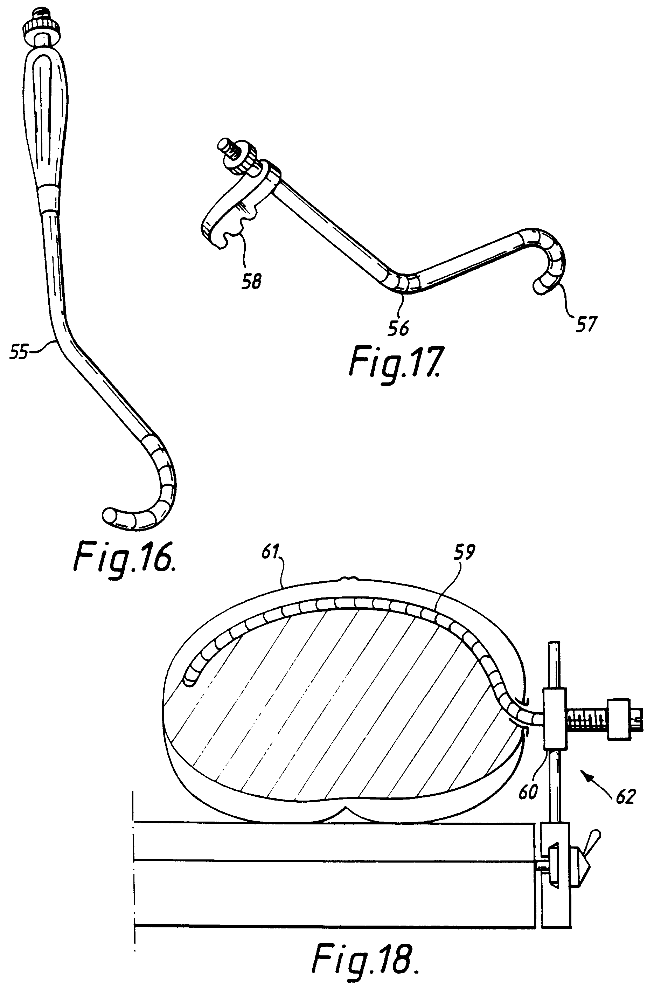

Figure 16 shows a retractor having a permanently bent section 55. Figure 17 shows a retractor which can have a bent section 56 at an intermediate portion in addition to the bent retractor or pusher 57 at the end of the instrument. The instrument of Figure 17 also has an offset handle which will remain outside of the body cavity and which is of particular use in pelvic surgery.

In endoscopic surgery the cavity between the body wall and the organs is created and maintained as a result of a gas pressure. However, it is a considerable problem to try and maintain sufficient pressure as the camera and instruments are being inserted or removed. Figure 18 shows the use of an artificial "rib" 59 which is inserted through an opening 60 at one side of the body and extends across the body within the cavity and holds the wall 61 away from the organs.

The rib 59 can be inserted in a generally straight direction and then tightened to the position shown to raise the wall away from the organs. Alternatively or additionally, when the rib is locked in the curved configuration shown it can then be manoeuvred to raise the body wall clear of the organs.

More than one rib may be provided to raise a whole region of body wall, in which case the ribs may be generally parallel but spaced from each other.

Retaining means 62 may be provided to hold the ribs in the required position during an operation.

With such a rib or ribs, the operation may be performed with low or no excess gas pressure within the cavity.

With the illustrated rib, four openings may be equispaced around the longitudinal extent of each segment of the retractor with a pair of wires passing through those openings and around the end of the rib. Having an extent of wire through each of the openings increases the strength of the rib, particularly where the wires are under tension and retains the rib in the bent configuration with more rigidity than is achieved with a single wire passing through diametrically opposite openings.

In Figure 19 the end of the instrument includes a pair of blade members 49 either or both of which can be pivoted about an axis 50 to move the blade members towards each other either to cut, or to compact the end to allow insertion and removal through the narrow body wall passage. The central filament 48 actuates the or each blade member at the required time. Release of tension allows or causes the blades to return to the position illustrated. Return of the filament 48 may cause or assist in divergence of the blade member or, alternatively or additionally resilient means such as a spring may cause or assist in the blade members moving relative to each other to a closed or open position.

In the embodiment shown in Figures 20 and 21 a needle holder 51 and a forcep grasper 52 respectively are shown at the end of the instruments. A pivotal member 53 or 54 is movable about the axis 50 to enable a needle or forcep to be gripped as required. Movement and maintaining the pivotal members 53 or 54 is as described above with regard to the relative pivotal movement of the blade members in Figure 19.

Figure 22 shows an instrument having a pair of blade members 49, as shown in Figure 19. The segments 63 are angled relative to each other such that after the wire has been tightened to cause the segments to extend in a curve, the blade members extend in substantially the opposite direction to that in which they extended during insertion through the body wall. The blade members are operable by tightening a flexible cord or filament which extends through a central opening in each segment, as previously described.

It will be appreciated that the blade members could be replaced by other tools such as forcep or needle holders which return to or face back towards the person holding the remote end of the instrument.

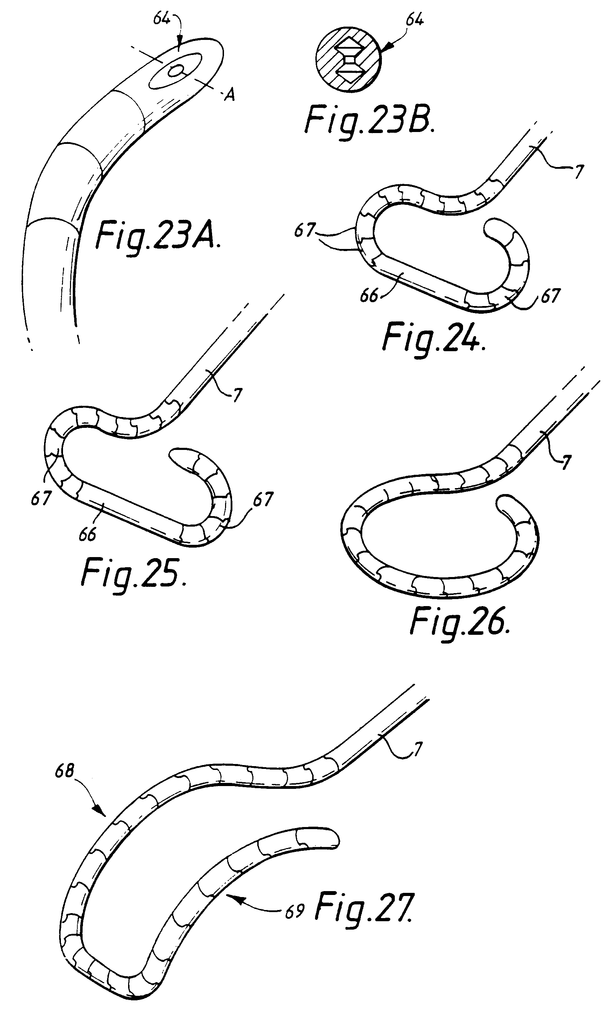

Figures 23A and 23B show the end of an instrument which may be curved after insertion into a body cavity being provided with an eye or formination 64 for ligature or thread access or return.

As shown in Figures 24 to 28, the segments may be arranged to provide almost any shape which is desired when the wires within the segments are tightened, and yet still permit relatively easy insertion into a body and retraction therefrom of the device in a straight line when the wires are relaxed.

In Figure 24 a "lozenge" shape is provided by having an elongate segment 66 located between suitably arranged segments 67. The "lozenge" in Figure 25, instead of being substantially flat as in Figure 24, extends at a 45° angle to the extent of the rod 7. Figure 26 shows a ring which may be either angled, relative to the rod 7, or flat with respect to that rod.

Figure 27 shows segments which have been orientated to provide a first curved section 68 and a second spaced substantially parallel curved section 69.

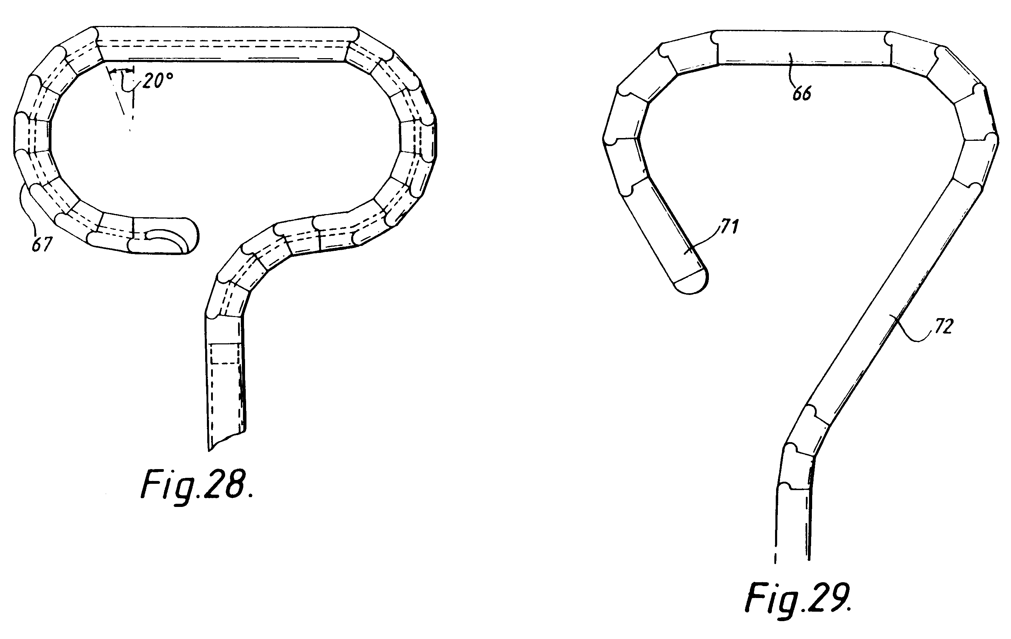

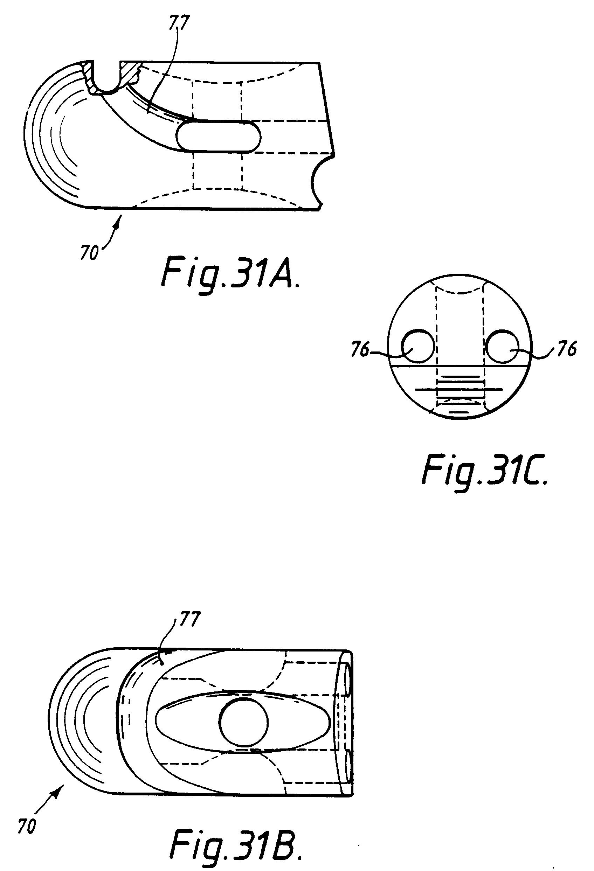

In Figure 28, which shows in detail the flat lozenge of Figure 24, the 20° angle of the segments 67 and the angle at the ends of the elongate segment 66 can be more clearly viewed, as can the end segment 70, shown more clearly in Figures 31A to 31C.

Figure 29 is a configuration similar to that shown in Figure 26 with the exception that an end straight section 71 is included spaced from one side of the elongate segment 66, with an elongate section 72 being located on the other side of the segment 66 but spaced therefrom.

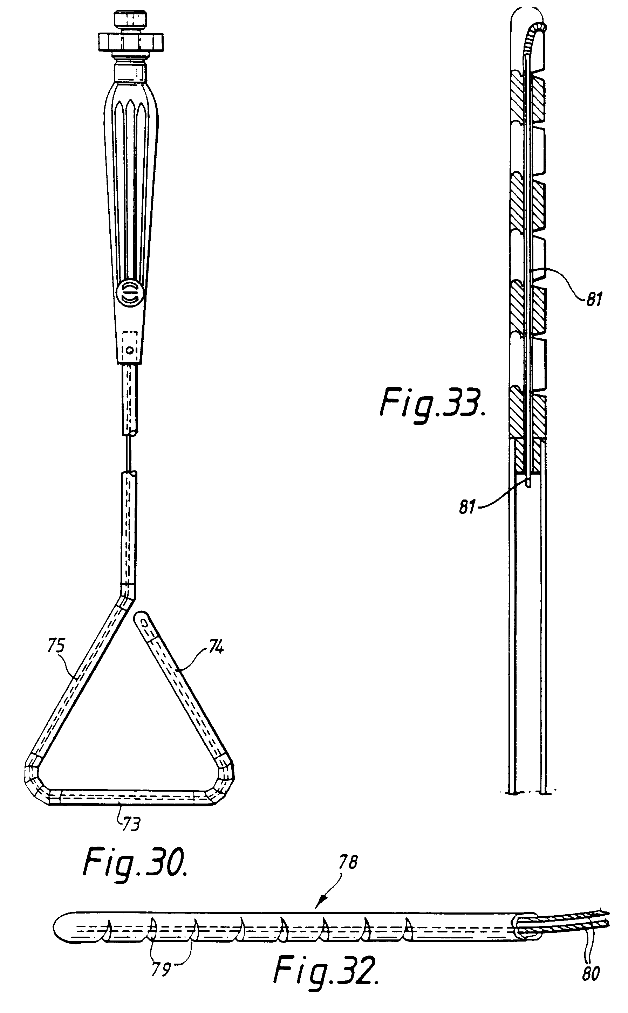

Figure 30 is a view of a retractor having a similar end configuration to that shown in Figure 29 with the exception that the straight sections 73 and 74 are of the same length but slightly shorter than the section 75 in order to provide the triangular shaped end.

Figures 31A to 31C show the details of the end segment 70. That segment is provided with two passageways 76 through which the extents of wire pass, and a channel 77, open to the exterior of the segment and in communication with the passageways 76. The wire lies in the smooth passageway when the segment is assembled to the end of the retractor. The end segment 70 may be used with any of the instruments illustrated.

Figure 32 illustrates an alternative way of forming a retractor with segments. The segments are formed by taking a length of plastics 78 and cutting notches 79 from one side towards the other. However, the notches do not extend through to the other side and the remaining portion of plastics acts as a hinge about which adjacent segments can pivot. The location, angle and extent of the notches can be adjusted as required to enable the retractor to take up any shape upon tightening of the wire 80. With this embodiment it can be seen that the segments are integral with each other. If desired the plastics can be moulded with the segments being formed in the mould.

Figure 33 shows the inclusion of a "memory metal" core 81. That core 81 is located between the two extents of the wire 82, as shown in the detail drawing, and is free to slide through the segments.

The core 81 bends when the segments are caused to bend but "remembers" the original orientation that the core occupies, as shown in Figure 33. Accordingly when the wire is tightened and the instrument is caused to bend, the wire also bends. However, the wire tends to urge the segments back to the position shown. Thus, even when the segments are not fully tightened against each other the force of the wire and the counter force of the core may be sufficient to hold the instrument sufficiently rigid to allow the instrument to be used in an operation.

When the tension in the wire is relaxed, the core returns the segments to the position shown and acts to retain them in that position.

The provision of the core also assists in maintaining the rigidity of the instrument during insertion through the wall of a patient and through the body of a patient.

The core 81 may be included in any of the instruments herein referred to in order to achieve the same or similar effect as those described in relation to Figure 33.

Any of the instruments described may have a complete or partial waterproof sleeve or plastic sheath, if desired. The sleeve or sheath may assist in preventing snagging of the instrument during insertion, or contamination of the body or instrument and may assist in flexing the instrument back towards a straight configuration when the tension in the wires is released.

In all of the herein described embodiments an instrument which is able to be inserted through a tube and is also able to take up a configuration in which it extends beyond the region bounded by the tube used to introduce it is provided, with a rigid configuration if necessary.

## Claims

1. A surgical instrument (1) including an elongate portion (2,7) arranged, in use, to be inserted through a restricted opening into a body cavity, the elongate portion (2) comprising a plurality of segments (3,29,32,35,63,66,67,71,73,74,75,70,78) movable relative to each other from a first to a second position whereby, when movement of the segments occurs from the first to the second position the relative direction in which a part of the elongate portion extends when compared to another part of the elongate portion is altered characterised in that, in the second position of the segments, further movement of the segments away from the first position is inhibited by abutments provided on the segments.

2. A surgical instrument as claimed in Claim 1 in which further movement of the segments away from the first position is inhibited by abutment of adjacent segments.

3. A surgical instrument as claimed in Claim 1 or 2 in which, when the segments are urged towards each other from the first to the second position, the force urging the segments towards each other acts between two locations where a segment contacts an abutment.

4. A surgical instrument as claimed in any preceding claim in which the segments are comprised by separate pieces.

5. A surgical instrument as claimed in any of Claims 1 to 3 in which the segments are comprised by adjacent portions which are integral with each other.

6. A surgical instrument as claimed in Claim 5 in which the segments are integrally moulded.

7. A surgical instrument as claimed in Claim 5 or 6 in which the segments are formed by removing a portion of integral material.

8. A surgical instrument as claimed in any preceding claim in which the segments are guided during at least part of their movement from the first to the second position.

9. A surgical instrument as claimed in any preceding claim in which segments cooperate with each other when in the second position to inhibit movement relative to each other in at least one direction transverse to the longitudinal extent of the elongate member.

10. A surgical instrument as claimed in any preceding claim including at least three segments movable relative to each other from the first position to the second position whereby movement of those segments from the first to the second position causes relative movement between an intermediate segment and an adjacent segment on one side to be in a first direction relative to the longitudinal extent of the elongate portion and relative movement between the intermediate segment and an adjacent segment on the other side to be in a second direction extending at an angle to both the first direction and the longitudinal extent of the elongate portion.

11. A surgical instrument as claimed in any preceding claim in which, in the first position, the elongate portion extends in a generally straight direction.

12. A surgical instrument as claimed in any preceding claim in which in the second position the elongate portion curves through more than 45° or 90° or 120° or in the region of 180° or more.

13. A surgical instrument as claimed in any preceding claim in which, in the second position, the elongate portion forms a constriction.

14. A surgical instrument as claimed in any preceding claim in which the segments are arranged to be biased towards each other in order to move from the first to the second position.

15. A surgical instrument as claimed in Claim 14 in which the segments are biased towards each other by a flexible member.

16. A surgical instrument as claimed in Claim 15 in which the flexible member extends through the segments.

17. A surgical instrument as claimed in Claim 15 or 16 in which the flexible member is arranged to be caused to be tensioned by control means located outside of a body when, in use, the segments are located within a body.

18. A surgical instrument as claimed in any preceding claim in which the cross sectional area of the instrument, in a direction perpendicular to the extent of the longitudinal portion, remains largely unchanged along the longitudinal extent in both the first and second positions.

19. A surgical instrument as claimed in any preceding claim in which segments are located at an intermediate portion along the elongate portion.

20. A surgical instrument as claimed in any preceding claim in which segments are located towards the end of the elongate portion.

21. A surgical instrument as claimed in any preceding claim in which an extent of the elongate portion is covered with a protective layer.

22. A surgical instrument as claimed in Claim 21 in which the extent of the elongate portion which is covered by the protective layer is substantially the complete extent.

23. A surgical instrument as claimed in any preceding claim including a mechanism connected to one of the segments on the elongate portion.

24. A surgical instrument as claimed in any preceding claim in which substantially the complete extent of the elongate portion is comprised by segments movable relative to each other from the first to the second position.

25. A surgical instrument as claimed in any preceding claim including an elongate resilient member arranged to act on adjacent segments in order to urge them towards a particular position.

26. A surgical instrument as claimed in Claim 25 in which the resilient member is arranged to urge adjacent segments towards the first position.

27. A surgical instrument as claimed in Claim 25 or 26 in which the resilient member is arranged to enable the instrument to have sufficient stiffness to enable use at a position in which the segments are away from the first position, in a second position which is away from a limit position.

## Patentansprüche

1. Chirurgisches Instrument (1) mit einem langgestreckten Teil (2, 7), der dazu dient, beim Gebrauch durch eine begrenzte Öffnung in eine Körperhöhlung eingeführt zu werden und der eine Mehrzahl von Segmenten (3, 29, 32, 35, 63, 66, 67, 71, 73, 74, 75, 70, 78) aufweist, die von einer ersten in eine zweite Position relativ zueinander bewegbar sind, bei welcher Bewegung die relative Richtung, in welcher sich ein Teil des langgestreckten Teiles erstreckt, verglichen mit einem anderen Teil des langgestreckten Teiles, verändert wird, dadurch gekennzeichnet, daß eine weitere Bewegung der Segmente in deren zweiter Position von der ersten Position aus durch Anschläge unterbunden wird, die an den Segmenten vorgesehen sind.

2. Chirurgisches Instrument nach Anspruch 1, dadurch gekennzeichnet, daß eine weitere Bewegung der Segmente hinweg von der ersten Bewegung durch Anschläge einander benachbarter Segmente unterbunden wird.

3. Chirurgisches Instrument nach Anspruch 1 oder 2, wobei dann, wenn die Segmente von der ersten in die zweite Position gegeneinandergedrückt werden, die die Segmente gegeneinander drückende Kraft zwischen jenen Orten wirkt, an welchen ein Segment einen Anschlag berührt.

4. Chirurgisches Instrument nach einem der vorausgegangenen Ansprüche, wobei die Segmente aus getrennten Teilen bestehen.

5. Chirurgisches Instrument nach einem der Ansprüche 1 bis 3, wobei die Segmente einander benachbarte Teile umfassen, die einteilig sind.

6. Chirurgisches Instrument nach Anspruch 5, wobei die Segmente einteilig geformt sind.

7. Chirurgisches Instrument nach Anspruch 5 oder 6, wobei die Segmente dadurch geformt sind, daß ein Teil aus dem vollen Material weggenommen wird.

8. Chirurgisches Instrument nach einem der vorausgegangenen Ansprüche, wobei die Segmente während wenigstens eines Teiles ihrer Bewegung von der ersten zur zweiten Position geführt sind.

9. Chirurgisches Instrument nach einem der vorausgegangenen Ansprüche, wobei die Segmente dann miteinander zusammenarbeiten, wenn sie sich in der zweiten Position befinden, um eine Bewegung relativ zueinander in wenigstens einer Richtung quer zur Längsrichtung des langgestreckten Elementes unterbunden wird.

10. Chirurgisches Instrument nach einem der vorausgegangenen Ansprüche, mit wenigstens drei Segmenten, die relativ zueinander bewegbar sind von einer ersten zu einer zweiten Position, wobei die genannte Bewegung jene Elemente von der ersten zur zweiten Position eine Relativbewegung zwischen einem Zwischensegment und einem benachbarten Segment auf einer Seite hervorruft, in einer ersten Richtung relativ zur Längserstreckung des langgestreckten Teiles sowie eine Relativbewegung zwischen dem Zwischensegment und einem benachbarten Segment auf der anderen Seite in einer zweiten Richtung, die sich unter einem Winkel zur ersten Richtung und zur Längserstreckung des langgestreckten Teiles erstreckt.

11. Chirurgisches Instrument nach einem der vorausgegangenen Ansprüche, wobei sich das langgestreckte Element in der ersten Position im wesentlichen geradlinig erstreckt.

12. Chirurgisches Instrument nach einem der vorausgegangenen Ansprüche, wobei sich das langgestreckte Element in der zweiten Position um mehr als 45 oder 90 oder 120° oder im Bereich von 180° oder mehr krümmt.

13. Chirurgisches Instrument nach einem der vorausgegangenen Ansprüche, wobei das langgestreckte Teil in der zweiten Position eine Einschnürung bildet.

14. Chirurgisches Instrument nach einem der vorausgegangenen Ansprüche, wobei die Segmente derart angeordnet sind, daß sie gegeneinandergedrückt werden, so daß sie sich von der ersten in die zweite Position bewegen.

15. Chirurgisches Instrument nach Anspruch 14, wobei die Segmente durch ein flexibles Element gegeneinandergedrückt werden.

16. Chirurgisches Instrument nach Anspruch 15, wobei sich das flexible Element durch die Segmente hindurcherstreckt.

17. Chirurgisches Instrument nach Anspruch 15 oder 16, wobei das flexible Element derart angeordnet ist, daß es durch ein Steuerelement außerhalb des Körpers dann gesteuert wird, wenn sich die Segmente beim Gebrauch innerhalb des Körpers befinden.

18. Chirurgisches Instrument nach einem der vorausgegangenen Ansprüche, wobei ein Querschnittsbereich des Instrumentes in einer zur Erstreckung des langgestreckten Teiles entlang der Längserstreckung in der ersten und in der zweiten Position weitgehend unverändert bleibt.

19. Chirurgisches Instrument nach einem der vorausgegangenen Ansprüche, wobei die Segmente in einem Zwischenteil entlang dem langgestreckten Teil angeordnet sind.

20. Chirurgisches Instrument nach einem der vorausgegangenen Ansprüche, wobei die Segmente gegen das Ende des langgestreckten Teiles hin angeordnet sind.

21. Chirurgisches Instrument nach einem der vorausgegangenen Ansprüche, wobei ein Abschnitt des langgestreckten Teiles von einer Schutzschicht bedeckt ist.

22. Chirurgisches Instrument nach Anspruch 21, wobei der mit der Schutzschicht bedeckte Teil des langgestreckten Teiles im wesentlichen dessen gesamte Länge umfaßt.

23. Chirurgisches Instrument nach einem der vorausgegangenen Ansprüche, mit einem Mechanismus, der an eines der Segmente des langgestreckten Teiles angeschlossen ist.

24. Chirurgisches Instrument nach einem der vorausgegangenen Ansprüche, wobei im wesentlichen die gesamte Länge des langgestreckten Teiles von Segmenten gebildet ist, die von der ersten zur zweiten Position relativ zueinander beweglich sind.

25. Chirurgisches Instrument nach einem der vorausgegangenen Ansprüche, umfassend ein langgestrecktes elastisches Element, das derart angeordnet ist, daß es auf einander benachbarte Segmente einwirkt, um diese in eine bestimmte Position zu drücken.

26. Chirurgisches Instrument nach Anspruch 25, wobei das elastische Element derart angeordnet ist, daß es einander benachbarte Elemente in die erste Position drückt.

27. Chirurgisches Instrument nach Anspruch 25 oder 26, wobei das elastische Element derart angeordnet ist, daß es dem Instrument genügend Steifigkeit verleiht, um dessen Gebrauch in einer Position zu erlauben, in welcher die Segmente fern der ersten Position sind in einer zweiten Position, die fern von einer Grenzposition ist.

## Revendications

1. Instrument chirurgical (1) comprenant une partie allongée (2, 7) adaptée, à l'utilisation, pour être insérée par une ouverture limitée pratiquée dans une cavité corporelle, la partie allongée (2) comprenant une pluralité de segments (3, 29, 32, 35, 63, 66, 67, 71, 73, 74, 75, 70, 78) mobiles les uns par rapport aux autres d'une première à une seconde position, au moyen desquels, lorsque se produit le mouvement des segments de la première à la seconde position, la direction relative dans laquelle une partie de la partie allongée s'étend comparée à une autre partie de la partie allongée est altérée, ***caractérisé en ce que***, dans la seconde position des segments, un plus ample mouvement d'éloignement des segments vis-à-vis de la première position est empêché par des aboutements prévus sur les segments.

2. Instrument chirurgical selon la Revendication 1, dans lequel le plus ample mouvement d'éloignement des segments vis-à-vis de la première position est empêché par l'aboutement de segments adjacents.

3. Instrument chirurgical selon la Revendication 1 ou 2, dans lequel, lorsque les segments sont forcés les uns vers les autres de la première à la seconde position, la force poussant les segments les uns vers les autres agit entre deux emplacements où un segment rentre en contact avec un aboutement.

4. Instrument chirurgical selon l'une quelconque des Revendications précédentes, dans lequel les segments sont constitués de pièces distinctes.

5. Instrument chirurgical selon l'une quelconque des Revendications 1 à 3, dans lequel les segments sont constitués de portions adjacentes qui font partie intégrante les unes des autres.

6. Instrument chirurgical selon la Revendication 5, dans lequel les segments sont moulés d'une seule pièce.

7. Instrument chirurgical selon la Revendication 5 ou 6, dans lequel les segments sont formés en enlevant une portion du matériau d'une seule pièce.

8. Instrument chirurgical selon l'une quelconque des Revendications précédentes, dans lequel les segments sont guidés pendant au moins une partie de leur déplacement de la première à la seconde position.

9. Instrument chirurgical selon l'une quelconque des Revendications précédentes, dans lequel les segments coopèrent entre eux lorsqu'ils sont dans la seconde position afin d'empêcher le mouvement les uns par rapport aux autres dans au moins une direction transversale à l'étendue longitudinale de l'élément allongé.

10. Instrument chirurgical selon l'une quelconque des Revendications précédentes comprenant au moins trois segments mobiles les uns par rapport aux autres de la première position à la seconde position, au moyen desquels le mouvement de ces segments de la première vers la seconde position provoque un mouvement relatif entre un segment intermédiaire et un segment adjacent d'un premier côté vers une première direction par rapport à l'étendue longitudinale de la partie allongée et un mouvement relatif entre le segment intermédiaire et un segment adjacent de l'autre côté vers une seconde direction s'étendant en angle à la fois par rapport à la première direction et par rapport à l'étendue longitudinale de la partie allongée.

11. Instrument chirurgical selon l'une quelconque des Revendications précédentes, dans lequel, dans la première position, la partie allongée s'étend dans une direction globalement droite.

12. Instrument chirurgical selon l'une quelconque des Revendications précédentes, dans lequel, dans la seconde position, la partie allongée s'incurve de plus de 45°, ou 90°, ou 120° ou dans la région de 180° ou plus.

13. Instrument chirurgical selon l'une quelconque des Revendications précédentes, dans lequel, dans la seconde position, la partie allongée forme un rétrécissement.

14. Instrument chirurgical selon l'une quelconque des Revendications précédentes, dans lequel les segments sont prévus pour être déportés obliquement les uns vers les autres de la première vers la seconde position.

15. Instrument chirurgical selon la Revendication 14, dans lequel les segments sont déportés obliquement les uns vers les autres par un élément flexible.

16. Instrument chirurgical selon la Revendication 15, dans lequel l'élément flexible s'étend au travers des segments.

17. Instrument chirurgical selon la Revendication 15 ou 16, dans lequel l'élément flexible est prévu pour que soit provoquée sa mise sous tension par des moyens de commande situés à l'extérieur d'un corps lorsque, à l'utilisation, les segments se trouvent à l'intérieur du corps.

18. Instrument chirurgical selon l'une quelconque des Revendications précédentes, dans lequel l'aire transversale de l'instrument, dans une direction perpendiculaire à l'étendue de la partie longitudinale, demeure largement inchangée tout le long de l'étendue longitudinale à la fois dans la première et la seconde positions.

19. Instrument chirurgical selon l'une quelconque des Revendications précédentes, dans lequel les segments sont situés sur une portion intermédiaire de la partie allongée.

20. Instrument chirurgical selon l'une quelconque des Revendications précédentes, dans lequel les segments sont situés vers l'extrémité de la partie allongée.

21. Instrument chirurgical selon l'une quelconque des Revendications précédentes, dans lequel une longueur de la partie allongée est recouverte d'une couche protectrice.

22. Instrument chirurgical selon la Revendication précédente, dans lequel la longueur de la partie allongée qui est recouverte d'une couche protectrice est substantiellement la longueur totale.

23. Instrument chirurgical selon l'une quelconque des Revendications précédentes, comportant un mécanisme raccordé à l'un des segments sur la portion allongée.

24. Instrument chirurgical selon l'une quelconque des Revendications précédentes, dans lequel substantiellement la totalité de la longueur de la partie allongée est composée de segments mobiles les uns par rapport aux autres de la première à la seconde position.

25. Instrument chirurgical selon l'une quelconque des Revendications précédentes, comportant un élément élastique allongé prévu pour agir sur des segments adjacents de manière à les forcer en direction d'une position particulière.

26. Instrument chirurgical selon la Revendication 25, dans lequel l'élément élastique est prévu pour forcer des segments adjacents vers la première position.

27. Instrument chirurgical selon la Revendication 25 ou 26, dans lequel l'élément élastique est prévu pour permettre à l'instrument d'avoir une rigidité suffisante pour permettre une utilisation dans une position pour laquelle les segments sont éloignés de la première position, dans une seconde position qui est éloignée d'une position limite.
